# EUROPEAN PATENT SPECIFICATION

(11) **EP 1 207 736 B1**
(45) Date of publication and mention of the grant of the patent: **27.08.2003**
(21) Application number: 00929726.8
(22) Date of filing: 17.05.2000
(51) Int. Cl.: A01D 43/00, C12P 21/00, C07K 14/415

(54) **METHOD FOR INDUCTION SYNTHESIS OF HEAT STRESS PROTEINS (HSP) & PRODUCTION FROM HERBACEOUS PLANTS**
VERFAHREN ZUR INDUKTION DER SYNTHESE VON HITZESCHOCK-PROTEINEN (HSP) UND HERSTELLUNG AUS GRÄSERN
PROCEDES D'INDUCTION DE LA SYNTHESE DES PROTEINES SUBISSANT UN STRESS THERMIQUE (HSP) ET PROCEDE DE PRODUCTION A PARTIR DE PLANTES HERBACEES

(43) Date of publication of application: 29.05.2002
(73) Proprietor: BRT GEORGIA Ltd., Tbilisi (GE)
(72) Inventor: JALIASHVILI, Tengiz, A., Tbilisi, 380007 (GE); JALIASHVILI, Nato, T., Tbilisi, 380007 (GE)
(74) Representative: Sternagel, Fleischer, Godemeyer & Partner Patentanwälte
(86) International application number: GE0000003
(87) International publication number: WO00070932

(56) References cited:
- FERULLO JEAN-MARC ET AL: "Post-harvest alteration of in vitro translatable mRNA population in alfalfa (Medicago sativa L.)." CROP SCIENCE, vol. 36, no. 4, 1996, pages 1011-1016, XP002146910 ISSN: 0011-183X

## Description

### Technical Field

The application relates generally to the rationalization of use natural resources, especially herbaceous plants for production high quality feed & full spectrum of HSP families, on which there are permanently increasing demand in different industries.

### Backgound Art

### HSP induction & synthesis in plants.

It was well established that, any intact & fresh plants' leaves & stems after short time expose to heat shock may well develop heat-tolerance phenomenon via increase synthesis of HSP. The mechanism of heat stress sensitive genes induction by heat & increase synthesis of HSP is greatly resemble as in animal & plant cells, so in microorganisms, too. Furthermore, there are considerable structural (amino acids sequence) homology between HSP families in spite of its origin (Nover 1991; Morimoto et al 1990;

Neumann et al 1989; McKenzie et al. 1988; Jindal et al. 1989). Plant kingdom respond to temperature arise above physiological optimum by opening stomata to enhance evaporation water from the soil, but in the case of water deficit in soil - by closing stomata, that is reason rapid increase of leaves temperature above the air temperature, what is the reason induction heat sensible stress genes & synthesis of HSP in field grown plants. Stomata close itself increase photo-respiration, light-induced high energy state blocking & enhance leaves' temperature via Endo-Biochemical Exothermic Heat (EBEH). In order to prevent "burning" fresh harvested herbaceous plants by EBEH, while harvest--dropped on ground, there is common recommendation to ventilate hay by overturning. Stomata close in plant cells involves anaerobic or desiccation or osmotic stresses & stress related conversions e.g. lactate to ethanol.

In spite of crossing several stresses in field condition, heat sensible genes response to heat stress is far more faster than to any other type of stress factors. Furthermore, only heat stress factor is highly specific & able to switch on synthesis full spectrum of HSP families, as constitutive HSP, so transit HSP, too (Burke at al, 1985; Burke 1990; Kimpel, Key 1985; Levitt 1980; Singla et al 1997; Pollock 1993; Petersen 1990).

Partial miss-folding 3D-structure of thermal labile proteins & accumulation abnormal proteins inside of cells under heat & other stress conditions, has considered as the most strong & swift internal inductor of HSP synthesis, too (Hoogenraad 1996).

There is neediness to underline, that only intact & fresh plant's cells able sense external or internal heat stress & transfer heat signal inside of cells via induction of heat sensible genes transcription & increase synthesis full spectrum of HSP families. As it is common transit nature of synthesis for almost HSP families, so content of HSP gradually declines in all cells after heat stress. At non heat stress condition plants are synthesize none or very small quantity of some HSP families. Moreover, increase synthesis of HSP in the case of heat stress condition, while only short period just after harvesting herbaceous plants. It is worth to mark out dramatic decrease ability of heat sensible genes to synthesize HSP just after herbaceous plants processing as commercial feed: either pellet, or hay or silage or silos.

None of exist in EU & USA harvesting & feed-pellet manufacturing agro-technologies without rationalization may be used for HSP induction & production from herbaceous plants.

That is the reason urgent necessity of rationalization exist agro-technologies in order to manage production of full spectrum HSP families on the base of only fresh herbaceous plants, as the cheapest natural resource. In order to outline properly integration of HSP induction in field grown herbaceous plants, with improvement of feed's quality, there is need to mark out following non-rationality of harvesting agro-technologies on the example of alfalfa: 1-with chopping directly in field just harvested alfalfa, which:
- loose of alfalfa cytoplasm contain as HSP, so nutrient, too;
- decrease as chaperone function of HSP, so feed proteins' efficiency via its' cross react with oxygen & phenols (Pierpoint 1983; Anderson 1968);
2-with dropping on ground of just harvested alfalfa, which:
- increase dramatically soil pathogens & mould in fresh bio-mass of alfalfa & plus complicate production of HSP.

### Disclosure of the Invention

### Summary of The Invention of harvesting agro-technologies:

- Stop chopping in field just harvested fresh alfalfa;
- Stop dropping on ground just harvested fresh alfalfa;
- Harvest & Transport fresh alfalfa intact condition from field;
- Shorten maximally time between alfalfa's chop & feed process.

### Pellet-feed production from fresh alfalfa (chopped in field).

There are the following non-rationality just exist pellet-feed agro-technology used by France Lucerne producer annually ∼1 million tone of pellet from ∼4 million tone of fresh alfalfa.

According to economical analyses 1 tone pellet-feed production from ∼4 tone fresh harvested & chopped in field alfalfa needs either ∼350 kg coal (250kW/h=140FF) or ∼190 mi gas (1250kW/h=85FF). That is reason that energy cost for only water evaporation from 4 million tone chopped fresh alfalfa is almost 85million FF with gas or 140 million FF with coal, annually.

Furthermore, to evaporate almost 3 milliard liter water from ∼4 000 000 tone fresh alfalfa, in order to process ∼1 000 000 tone pellet needs either long time, or very high temperature for fastening drying process. France Lucerne has used hot air for pellet process via drying fresh alfalfa: at starting +850°C & with finalizing +125°C.

### Summary of The Invention pellet agro-technology:

- Stop "burning" fresh chopped alfalfa's all proteins & vitamins efficiency & chaperone function of HSP with +850°C hot hair during pellet processing;
- Stop "evaporate" alfalfa's cytoplasm water, which is resource for natural cash-products manufacturing with permanently increasing demand on each of it (food proteins, vivacious water, bio-minerals, HSP).
- Replace cost additive evaporation of alfalfa's cytoplasm water, with cost save press-extracting, which may decrease ∼50% energy consumption of pellet processing (from press-cake) & plus will give juice as resource for manufacturing such renewable bio-tech products as HSP, with multi-marketable potential (pharmacy, veterinary, cosmetics, fitness, feed industry, etc.).
- Conserve high nutrient quality of alfalfa in press-cake via anaerobic packing it or with natural probiotic (Lactobacilus & lactic acid) to decrease up to ∼80% energy needs for feed processing.

### Assumption of innovations:

Pellet processing directly from fresh alfalfa chopped in field, is non-rational & cost additive neoclassical agro-technology which solidly decreases quality of feed. That is reason of urgent need to replace exist pellet agro-technology's formula "one resource-one product" by cost save bio-rational technology formula "one resource-many products" via extraction cytoplasm for separate processing colossal bio-tech value of fresh alfalfa. Only in the case of refinement exist harvesting & pelting agro-technologies of alfalfa in EU & USA, it will be possible to use short-term renewable alfalfa & other herbaceous plants as cheapest natural resource for non-waste production full spectrum of HSP families & keep high quality of feed, too.

### Marketable advantages of plant derived HSP

Main advantages of plant derived HSP (in comparison with animal cells & microorganism derived HSP) are the following:
- Natural sufficiency of herbaceous plants to respond to heat stress by swift production full spectrum of HSP families:
- Chaperone ability of plant derived HSP almost all families, & especially the biggest orchestra of low molecular mass HSP;
- High ecological criteria: non-waste production, complete bio-degradability, no pollution, World-wide distribution & strong sustainability of herbaceous plants;
- Integration low profitable agriculture with high profitable bio-tech based industries such as: Life Sciences, Pharmacy, etc.;
- Satisfaction of permanently growing social demand on plant origin therapeutics & its low risk for human health for present & future generations.

Moreover, plant derived HSP, in addition to established heat stress tolerance (Vierling 1990-91, Boston et al, 1996), may carry natural therapeutics role in formation & keeping stamina of herbivores (Jaliashvili 1989,1997). According to this theory during grazing fresh herbaceous plants by livestock, undergo heat stress in digestive tract of herbivores (at 37°C), what induce synthesis of HSP in plant cells & increase its release from plant cells after digestion of fresh feed. Plant derived HSP well fascinate immune-status of herbivores, what may be base mysterious curing of livestock while graze growing fresh grass. Good support to theory, which is considering plant HSP as natural therapeutics may facts that 70% of first line of defense, so called antigen representing cells (APC), is localized in digestive tract of animals (Service 1994, Gaskins 1996) & all HSP families are immune-modulators.

Furthermore. remarkable homology between animal & plant cells pre-owned HSP families, arising alfalfa plant derived HSP as leading bio-tech products for wide use in pharmacy & cosmetics. This possibility has greatly increased especially after:
- description plant HSP ability to stabilize 3-D structure of different origin proteins (Boston et al.,1996, Jindal et al 1989),
- refinishing of HSP immune-modulation ability (van Eden, Young 1996: Cohen 1992) &
- application in pharmacy universal carrier ability of HSP almost families to carry antigens & enhance human defense potential against many diseases e.g. cancer, tumors, diabetes, etc., (Srivastava 1999-2000, Cohen 1992, van Eden, Young 1996).

Plant derived HSP families roles definition in creation & keeping stamina of herbivores is just Frontiers of Sciences.

### HSP - Chaperone Functions

Chaperone function of HSP mostly resolvable by: short- & long-term folding & stabilizing 3-D structures of enzymes or cellular receptors or DNA/RNA. or fascinating proteins transport across membranes, or resolving denature proteins or re-folding of miss-folded proteins, or immune-stimulation via transiting antigens to APC or via marking foreign proteins & accelerating its apoptosis (Hoogenraad 1996; Boston et al., 1996, Morimoto at al 1990; Almaguera et al 1995).

Molecular mechanism of HSP chaperone function, may be explained via widening the range of intermolecular hydrophobic interaction inside of heat labile proteins & stabilizing it's 3-D structure during bio-liquids hydrodynamic characteristics fluctuation (under heat stress conditions). Common function for all chaperone of HSP families may be molecular water reallocation inside of 3-D structure of heat labile proteins.

### Classification of Heat Stress Proteins (HSP):

by Neuman et al 1989; & Singla et al 1997.
by encoding in different chromosomes:
   - nuclear genome encoded;
   - non-nuclear genome encoded (chloroplast, mitochondria);
by molecular mass & homology
   - HSP 110 (95-110 kDa):
   - HSP 90 (80-95 kDa);
   - HSP 70 (63-79 kDa);
   - HSP 60 (53-62 kDa);
   - HSP 20 (10-30 kDa);
   - HSP 8.5 (ubiquitin).

There is a demand for a method for the large-scale production of full spectrum HSP families because of the high demand on each of it in different industries, such as veterinary, pharmacy, cosmetics, feed industry, fitness, etc.

Methods which characterizing and isolating small amounts of HSP from plants using gel electrophoresis are known DeRocher et al, (1991). Plant Physiol., 1991. v. 96, pages 1038 to 1047 describes the characterization of cDNAs and HSP and the expression of HSP during a heat shock of plants. To this end plants which are 8 to 9 days old are exposed to a heat shock by increasing the temperature of the growth space at a speed of 4°C per hour until the desired temperature of 30°C to 40°C has been reached. This temperature is maintained for 4 hours and is then again decreased at a speed of 4°C per hour until a temperature of about 22°C has been reached. The HSP are isolated from the resulting plant material by electrophoresis.

An investigation of the expression of HSP in plants is described in Plant Physiol., 1993, v.101, pages 1209 to 1216 (Hernandez, Vierling 1993). The plants are here again subjected to a heat shock by being exposed to an increase in temperature at a speed of 4°C per hour and by being subjected to a treatment at the maximum temperature for 4 hours. Samples are then taken from the leaves of said plants for protein analysis. These samples are put into plastic bags and stored on ice until the samples are further isolate HSP by gel electrophoresis.

Moreover, methods are known for producing HSP by as genetic engineering on the base of microorganisms, mainly E. coli, so chemically, mainly synthetic fragments of it. It is worth to mark out that non of these methods will give natural molecules of HSP with post-translation tremendous modifications, & which are well characterizing animal or plant cells species specificity. Bacteria/virus/fungi lack highly specialized sub-cellular systems (dictyosomes, endoplasmic reticulum /ER/), which are existing in plant/animal cells in order to modify & target own proteins on specific functions. So, dictyosomes & ER as unique systems of plant/animal's cells, where the hole orchestra of HSP target & ascend all kind of enzymes & receptors & peptide hormones functions. In spite of that fact that the question of genome determined species specificity stays Nature's mystery up to now,
there is already strong evidence, that HSP carry prime role in controlling it. That is reason that gene-engineering, almost E. coli & synthetic chemical methods have the drawback that they are troublesome, expensive & required post-translation modification to yield natural HSP, what do not able neither these methods.

All these discoveries on importance of post-translation molecular modification of proteins in dictyosomes & ER by HSP, has induced world bio-tech industries shifting & focusing slowly towards practically more valuable plant cells' genome. Plant cells priority - as natural factories has just proved by such bio-tech giant as Monsanto Inc.

It is therefore the object of the present invention to provide a method which can be carried out easily and at low costs and which yields full spectrum of HSP all families with post-translation modified natural structures & functions, too.

This patent declaration claims non-waste production of full spectrum of HSP families with post-translation modified natural structures from herbaceous plants, on the example of in field grown Medicago sativa (alfalfa, Lucerne). There was done new attempt to induce HSP synthesis in fresh harvested alfalfa by endo-biochemical exothermic heat (EBEH) as non-expensive source of energy for heat stress development in just harvested alfalfa. The base of induction & increase synthesis of HSP in just harvested & especially non-chopped fresh plant materials was considered:
- water supply canceling from soil & stomata close (in order to keep osmotic pressure intracellular spaces) & swift increase leaves temperature above air temperature on the base of EBEH, &
- partial miss-folding of heat labile proteins by EBEH, as one of the most strong inductor HSP synthesis in plant cells.

According to classical definition of exothermic reaction - heat is evolved & solutions' (or mixture) tend to contract, e.g. 75 300 calories per grams of water formed mixing two solutions of sodium hydroxide & with hydrogen chloride. This signification well suitable for plant materials, which tend to contract while endo-biochemical exothermic heat evolved.

This object is achieved by a method for the production of heat stress proteins (HSP) from fresh herbaceous plants, comprising the steps of:
a) harvesting the herbaceous plants,
b) heat stressing the herbaceous plant material directly before and/or during and/or after step a), wherein the expression of HSP derived from the full spectrum of the HSP families is induced in the plant material, and
c) extracting HSP from the harvested herbaceous plant material.

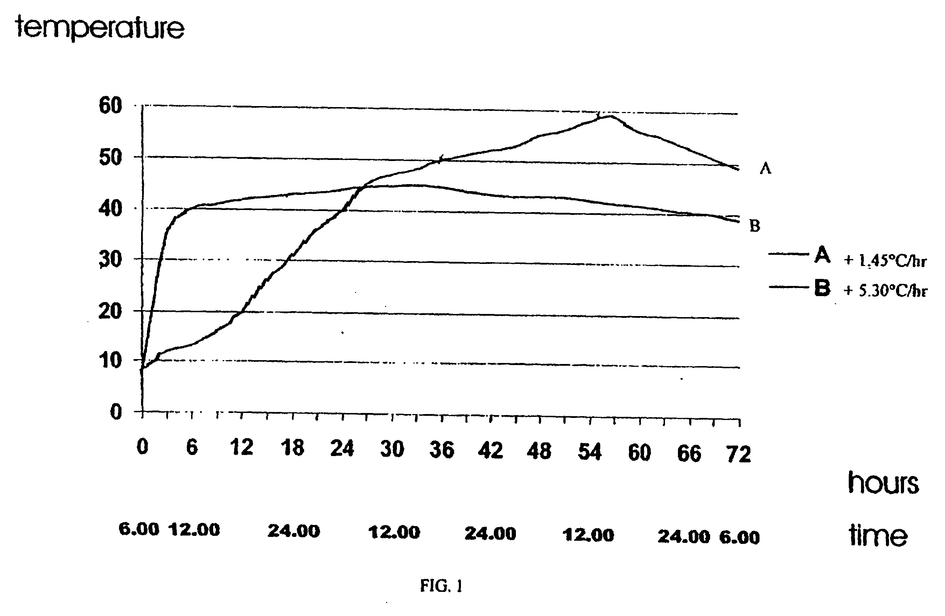

Primary focus on step (a) was rationalization harvesting fresh herbaceous plants in order to establish the most optimal condition for swift starting induction HSP synthesis & keep as chaperone ability of HSP so nutrient quality of feed via replacing exist agro-technologies with chopping or dropping on ground fresh alfalfa. Furthermore, as partial miss-fold heat labile proteins is the most swift way to induce heat stress genes, special construction was installed on harvester in order of achieve short term heat shock at early stage of harvesting. This construction is able use hot air from tractors' engine for establishment short-term heat shock just before harvesting alfalfa (see Fig 1). The degree of heat shock may be regulated (from 30°-up to 53°C). Such very short (from 30 second up to 3 min) heat shock is quite enough for labile proteins partial miss-fold e.g. RUBISCO (ribulose bisphosphate carboxylase) & heat stress gene stimulation & induction HSP synthesis in fresh harvested bio-mass of herbaceous plant.

It is worth to mark out that short term heat shock covers both as staying in soil, so harvesting parts of alfalfa, what makes the following integral benefices:
- will increase yield of HSP in harvested part of alfalfa, which almost all of it will obtain post translation modification, mostly all it with chaperone function & so will well preserve feed proteins nutrient quality in harvested plant material;
- will induce synthesis of HSP in non harvested part of alfalfa & establish stress acquired resistance (SAR) those part of alfalfa, which stayed in soil.

HSP synthesis stimulation in non harvested & stayed in soil part of alfalfa may well considered as new type of Intelligent Pest Management (IPM) for increase herbaceous plant self-defense ability after every harvest process.

Main focus on step (b) was done on maximization of yield spectrum of HSP families in harvested fresh alfalfa by Endo-Biochemical Exothermic Heat (EBEH), which able increase temperature of non-chopped plant material up to 45°C, while chopped plant material up to 60°C (see Fig.1, Line A & Line B).

Water deficiency just after harvesting of alfalfa, expedites stomata closing & development of endo-biochemical exothermic heat, what is the prime reason induction full spectrum of HSP families in leaves & stems cells of alfalfa.

Integration of short-term heat shock with long-term heat stress via EBEH able maximize spectrum of HSP families in harvested fresh alfalfa (see classification of HSP families).

Fig. 1 view of endo-biochemical exothermic heat development in harvested fresh alfalfa as non chopped so chopped conditions and

Fig. 2 view of spectrum HSP families induction and synthesis dependency on temperature change in harvested non chopped fresh alfalfa on the base of EBEH.

Within the meaning of the present invention the term "plant material" covers harvested fresh alfalfa both non chopped and chopped conditions.

Within the meaning of the present invention the term "heat stress proteins, with abbreviation HSP" stands for all those special type of proteins, whose intracellular concentration increases when plant cells is exposed to a heat stressful stimuli, or it is a protein showing at least 35% homology with representative of HSP any families or which possess chaperone ability.

Harvesting of the alfalfa in step (a) comprises yield of intact plant materials without chopping & dropping on ground & direct collecting & mildly pressing plant materials into bags or containers with common harvesting tractors (Pauli et al 1988: see harvesting tractors CMC F100, F120, enclose photo).

The plant materials that are usable in the method according to the invention may preferably be selected from any species of Medicago sativa (Alfalfa, Lucerne) & in addition from all herbaceous plants or from all those plant species, which suitable for leaf protein concentrate production described by Carlsson (1983) & Telek, Martin (1983) & Pirie (1971).

The induction heat stress sensible genes in the plant material & increase synthesis of full spectrum of HSP families was achieved mainly by heat shock before/or directly/or after step (a) & mainly during step (b). The heat stress of the plant material in step (b) of the method according to the invention can be carried out during and after step (a) to ensure the production of heat stress proteins full spectrum in the plant material during the whole harvesting operation and thereafter.

Preferably, however, the plant material is heat stressed after step (a) to simplify the method according to the invention.

Particularly preferably, heating after step (a) of the method according to the invention is carried out at least in part by exothermic heat, which based on endo-biochemical reaction of plant cells. As a result, the method of the invention can be carried out rational easily manner and ecologically well acceptable as no exogenous energy is needed for the heat stress development.

Exothermal heat stress can be effected by introducing the plant material into substantially airtight containers directly after step (a), preferably within about 1 hour, in particular within about 30 minutes, most preferably within 1 minute. After the containers have been closed, the temperature of the plant material inside the closed containers will rise at a speed of about 5°C per hour on account of anaerobic endo-biochemical reaction on the base of stomata close for the reason of water deficit in fresh harvested plant material. The temperature can here reach about 45°C in non chopped plant material at the most, while about 60°C in chopped plant materials. The plant material is thereby subjected to a short term heat shock and the partial denature of heat labile proteins (e.g. RUBISCO), to accelerate synthesis of heat stress proteins in the plant material.

Heat shock of the plant material directly before and/or during step (a) of the method according to the invention is preferably effected by using a radiator and/or blower.

Heat stress of the plant material in step (b) of the method according to the invention is preferably carried out to reach a temperature ranging from about 30°C to about 60°C.

Heat stress of the plant material directly before step (a) of the method according to the invention is preferably carried out at temperature range from about 40°C to about 60°C, in particular of about 45°C. Heating during and/or after step (a) is preferably carried out to reach a temperature ranging from about 30°C to about 45°C, in particular from about 35°C to about 40°C, most preferably from about 38°C to 40°C.

Heat stress of the plant material in step (b) of the method according to the invention is preferably carried out for a period of about 1 minute to about 75 hours.

When the plant material is heat shocked in step (a) of the method according to the invention directly before step (b), heat shock is preferably carried out for a period of about 1 second to about 1 minute, in particular for about 30 seconds. When heat stress is carried out in step (b) of the method according to the invention after step (a), heat stress is preferably carried out for up to about
75 hours, in particular up to 72 hours, more preferably about 1 hour to about 6 hours, and most preferably about 30 minute to about 3 hours.

The plant material may be chopped prior to heat stress after step (a), but the plant material is preferably not chopped prior to heat stress after step (a) to ensure an optimum condition for heat stress development & increase synthesis of HSP & prevent phenols cross react with as feed proteins, so HSP.

Preferably, the plant material may be compact via mild press for the purpose of endo-biochemical exothermal heat acceleration & equal distribution among plant materials after step (a).

Heat stress in step (b) of the method according to the invention can preferably be carried out at least in part using the waste heat of the engine of the harvester used in step (a), whereby to induce partial miss-folding heat labile proteins & reach swiftly threshold heat stress is remarkably accelerated synthesis of full spectrum HSP families when the method according to the invention is carried out.

The heat stress proteins are then extracted in step (c) of the method according to the invention from the plant material obtained after step (b).

Extraction heat stress proteins is carried out by employing conventional methods. For instance, the plant material obtained in step (b) is pressed in a horizontal press, optionally after chopping.

A press cake is thereby obtained, as well as juice from the alfalfa cells' cytoplasm content. The juice from alfalfa cells is subjected to a thermal or acid treatment and then centrifuged or time dependent precipitated. A HSP families are mainly soluble & contain in supernatant as well as in-soluble part, as part of HSP bound with precipitate material are thereby obtained. HSP is concentrated by way of cross-flow filtration of supernatant, resulting enrich fractions by only soluble total spectrum of HSP families.

HSP powder were obtained from total spectrum of HSP families enriched fractions with conventional methods, such as precipitation with acetone or vacuum drying under mild heat or deep freezing by liofilization.

Natural post-translated structures of HSP all families contained in the plant material are thereby obtained.

The method of the invention shall now be explained with reference to examples.

### Example 1:

Early in the morning, 400 kg alfalfa were harvested at an ambient temperature of 8°C to 10°C and without chopping divided four parallel 50 kg & with chopping two parallel 50 kg & inserted into textile (or plastic) bags.

The temperature was measured continuously during 72 hr in each bags. According the of EBEH development in fresh alfalfa bio-mass temperature rose of plant material at a speed of 5.3°C per hour in non chopped plant material (Fig. 1, line B), while in chopped plant material temperature rose at a speed of 1.45°C per hour (Fig. 1, line A).

The threshold temperature above 30°C was reached in non chopped plant materials after about 2 hours & 40°C for exerting the heat shock was reached after about 6 hours after harvesting (Fig. 1, line B), while in chopped plant materials arise according temperature: up 30°C -after about 18 hr, & up 40°C - after about 24hr after harvesting (Fig. 1, line A).

After 6 hours the temperature of the non chopped plant material did not rise any more, remaining at 42°C to 45°C for 72 hours, and then decreased at a slow rate (Fig.1. line B), while in chopped plant material temperature continue to arise up 60°C around 55hr, & only 60 hr start declining up to 50°C at 72 hour (Fig.1, line A).

At the same time in control plant material (50-50kg), which were lay out on the flour, temperature change was not significant neither in chopped nor in non chopped plant materials & stay at the range 15-22°C during 72 hr.

Alfalfa juice lose was significant in chopped plant material as in control one, which were lay out on the floor, so in experimental one, which was in bags.

There is a need some critical plant materials not less than 1kg, optimal 50kg or more to develop EBEH.

There is need of mild press on plant materials to develop HBEH.

### Example 2

Fresh harvested entire alfalfa around 400 kg without chopping was distributed into textile bags, as described in Example 1. After the temperature in the bags had reached 28°C to 30°C, 37°C to 39°C or 42°C to 45°C, samples of plant materials were taken, cut into pieces of a length of 3 to 5 cm, and then pressed immediately by horizontal press. After such a mechanical extraction from the fresh alfalfa cell's cytoplasm, a press cake was obtained that contained 60% of the total bio-mass, at a moisture content of around 60%, and also juice from alfalfa cytoplasm that contained 40% of the total bio-mass with about 10 to 12% of solids. The juice of the alfalfa cells was subjected either to a heat treatment at 95°C for 15 minutes or apple acid 51/50kg and then proteins' precipitate separated by centrifugation. The solids were separated from the supernatant, and it is assumed that the solids were mainly heat or acid labile proteins. The supernatant was concentrated 30 times by means of cross-flow filtration either using reverse osmotic (RO) or nano-filtration (NF) or ultra-filtration (UF) membranes according to the aims of HSP families production. RO or NF membranes able concentrate total spectrum of HSP families, while UF membranes separate HSP families e.g. low or high molecular mass HSP. In any case all obtained HSP families from fresh alfalfa cells considered as heat or acid stable, and their composition was determined according to common method e.g. by one- or two dimensional SDS gel electrophoresis or by HPLC (high pressure liquid chromatography) or immune-blotting described elsewhere by Hernandez, Vierling (1996), Neumann et al., (1989), Bettany (1996), Srivastava 2000.

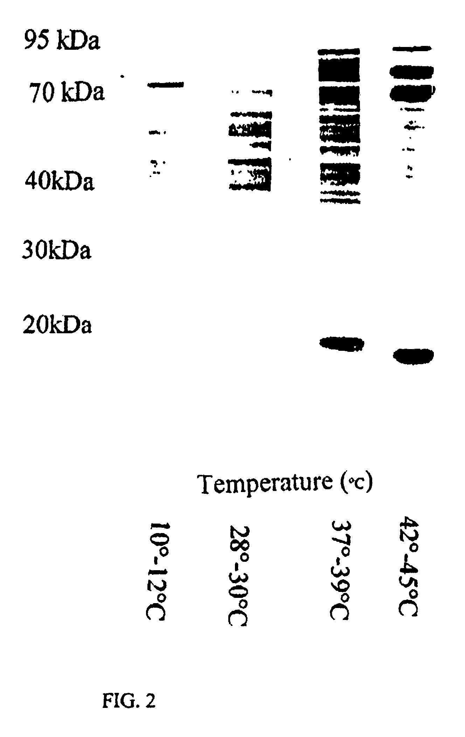

The view of obtained total spectrum of HSP families from fresh alfalfa grown in field was investigated by one-dimensional SDS gel electrophoresis are shown in Fig. 2.

As becomes apparent from Fig. 2, the content of spectrum HSP families in alfalfa cells is negligible at a temperature of 10°C to 12°C. When the temperature is rising to a range of from 28°C to 30°C, the production of the stress proteins also increases significantly, reaching a maximum at 37°C to 39°C. A further rise in temperature up to 42°C to 45°C, in turn, reduces the spectrum of HSP families. It may happen that the same spectrum of HSP families in alfalfa is maintained up to 20 hours in the case of exothermal heat stress due to endo-biochemical processes.

The total yield of full spectrum of HSP families is around 30 mg from the cytoplasm extracts of freshly harvested 1 kg alfalfa, non chopped, non dropped on ground & treated by heat stress which was created via endo-biochemical exothermic heat (EBEH).

## Claims

1. A method for the production of heat stress proteins (HSP) from fresh herbaceous plants, comprising the steps of:
a) harvesting the herbaceous plants,
b) heat stressing the herbaceous plant material directly before and/or during and/or after step a), wherein the expression of HSP derived from the full spectrum of the HSP families is induced in the plant material, and
c) extracting HSP from the harvested herbaceous plant material.

2. The method according to claim 1, wherein in step b) the heat stress carried out after step a) is resulting at least in part by use of exothermal heat, which is produced due to endo-biochemical reactions in the cells of the harvested herbaceous plant material.

3. The method according to claim 1 or 2, wherein the heat stress is carried out at a temperature ranging from about 30 °C to 60 °C before or after step a).

4. The method according to claim 3, wherein heat stress is carried out directly before step a) for a period of about 1 second to 1 minute, in particular for about 30 seconds.

5. The method according to any one of the preceding claims, wherein heat stress is carried out after step a) for up to 75 h.

6. The method according to any one of the preceding claims, wherein the heat stress is carried out at least in part by using the waste heat of the engine of the tractor-harvester.

7. The method according to any one of the preceding claims, wherein the plant material is chopped and macerated after step b) to increase the amount of juice containing soluble protein comprising HSP being derived from the full spectrum of the HSP families.

## Patentansprüche

1. Ein Verfahren zur Herstellung von Hitzestress-Proteinen (HSP) aus frischen krautartigen Pflanzen, umfassend die Schritte:
a) Ernten der krautartigen Pflanzen,
b) Hitze-stressen des krautartigen Pflanzenmaterials direkt vor und/oder während und/oder nach Schritt a), wobei die Expression der von der ganzen Bandbreite der HSP-Familien entstammenden HSP in dem pflanzlichen Material induziert wird, und
c) Extraktion der HSP aus dem abgeernteten krautartigen Pflanzenmaterial.

2. Das Verfahren nach Anspruch 1, **dadurch gekennzeichnet, dass** der Hitzestress in Schritt b), der nach Schritt a) durchgeführt wird, zumindest teilweise aus der Verwendung der exothermalen Wärme herrührt, die aufgrund der endo-biochemischen Reaktionen in den Zellen des geernteten krautartigen Pflanzenmaterials erzeugt wird.

3. Das Verfahren nach Anspruch 1 oder 2, **dadurch gekennzeichnet, dass** der Hitzestress bei einer Temperatur in einem Bereich von etwa 30 °C bis 60 °C vor oder nach Schritt a) durchgeführt wird.

4. Das Verfahren nach Anspruch 3, **dadurch gekennzeichnet, dass** der Hitzestress direkt vor Schritt a) für eine Zeitdauer von etwa 1 Sekunde bis zu 1 Minute, vorzugsweise für etwa 30 Sekunden durchgeführt wird.

5. Das Verfahren nach irgendeinem der vorherigen Ansprüche, **dadurch gekennzeichnet, dass** der Hitzestress nach Schritt a) für bis zu 75 Stunden durchgeführt wird.

6. Das Verfahren nach irgendeinem der vorherigen Ansprüche, **dadurch gekennzeichnet, dass** der Hitzestress zumindest teilweise unter Verwendung der Abwärme des Motors der Erntemaschine durchgeführt wird.

7. Das Verfahren nach irgendeinem der vorherigen Ansprüche, **dadurch gekennzeichnet, dass** das Pflanzenmaterial nach Schritt b) zerhackt und mazeriert wird, um die Menge des Saftes zu erhöhen, enthaltend lösliches Protein, das HSP von der ganzen Bandbreite der HSP-Familien umfasst.

## Revendications

1. Un procédé de production de protéines heat shock (HSP) à partir de plantes herbacées fraîches, comprenant les étapes :
a) récolter les plantes herbacées,
b) soumettre la matière de plantes herbacée à un stress thermique directement avant et/ou durant et/ou après l'étape a), l'expression des HSP dérivés de l'ensemble du spectre des familles HSP étant induite dans la matière de plantes, et
c) extraire les HSP de la matière de plantes herbacée et récoltée.

2. Le procédé selon la revendication 1, **caractérisé par le fait que** le stress thermique dans l'étape b) qui est réalisée après l'étape a) résulte au moins en partie de l'utilisation de la chaleur exothermique qui est générée en raison des réactions endo-biochimiques dans les cellules de la matière de plantes herbacée et récoltée.

3. Le procédé selon la revendication 1 ou 2, **caractérisé par le fait que** le stress thermique est effectué à une température comprise entre environ 30 °C et 60 °C avant ou après l'étape a).

4. Le procédé selon la revendication 3, **caractérisé par le fait que** le stress thermique est effectué directement avant l'étape a) pour une durée d'environ 1 seconde à 1 minute, de préférence pour 30 secondes à peu près.

5. Le procédé selon l'une quelconque des revendications précédentes, **caractérisé par le fait que** le stress thermique est effectué après l'étape a) pour une durée allant jusqu'à 75 heures.

6. Le procédé selon l'une quelconque des revendications précédentes, **caractérisé par le fait que** le stress thermique est effectué au moins en partie en utilisant la chaleur perdue du moteur de la moissonneuse.

7. Le procédé selon l'une quelconque des revendications précédentes, **caractérisé par le fait que** la matière de plantes est hachée et macérée après l'étape b) pour augmenter la quantité du jus contenant de la protéine soluble comprenant HSP de l'ensemble du spectre des familles HSP.
